# EUROPEAN PATENT APPLICATION

(11) **EP 1 559 792 A2**
(43) Date of publication of application: **03.08.2005**
(21) Application number: 04019589.3
(22) Date of filing: 18.08.2004
(51) Int. Cl.: C12Q 1/68, C12N 15/11, B01L 3/00

(54) **Gene-related RNAi transfection method**

(30) Priority: 11.09.2003 US 661005
(71) Applicant: EPPENDORF AG, 22339 Hamburg (DE)
(72) Inventor: Remacle, José, 5000 Malonne (BE); Bülow, Sven, 22395 Hamburg (DE)
(74) Representative: Becker Kurig Straus

(57) **Abstract**

The present invention relates to a novel method for determining the biological effect of a siRNA species in a cell. In particular, the present invention provides a method, wherein a support is provided, containing on predetermined locations thereof nucleic acids, representing or giving rise to a siRNA species. The support is covered by cells, into which the siRNA are taken up and exert their effect. Then the biological effect of the siRNA in the cell may be determined.

SEQUENCE LISTING

<110> Eppendorf AG

<120> Gene-related RNAi transfection method

<130> 80443 EP

<160> 5

<170> PatentIn version 3.1

<210> 1
<211> 20
<212> DNA
<213> artificial

<220>
<221> misc_feature
<222> (20)..(20)
<223> random nucleotide

<400> 1
taatacgact cactataggn 20

<210> 2
<211> 19
<212> DNA
<213> artificial

<400> 2
agaggaacag gatgccagc 19

<210> 3
<211> 23
<212> DNA

<213>  artificial

<400>  3
ttaaaaacag gtcacaacac taa                                              23

<210>  4

<211>  20

<212>  DNA

<213>  artificial

<400>  4
agcacagaag atgggaacac                                                  20

<210>  5

<211>  21

<212>  DNA

<213>  artificial

<400>  5
cagccacctc ttaaaagtat c                                                21

## Description

### Field of the invention

The present invention relates to a novel method for determining the biological effect of a short interfering RNA species in a cellular system. In particular, in the present invention use is made of a support containing on predetermined locations thereof nucleic acids, representing or giving rise to a short interfering RNA species. Cells are layered on the support and the short interfering RNA is taken up by the cells and exerts a biological effect. This biological effect of the short interfering RNA in the cell is determined. The present invention also relates to the evaluation of the impact of short interfering RNA on the effects of drugs or chemical compounds on biological processes.

### Description of the related art

The term RNA interference (RNAi) was coined after the discovery that injection of dsRNA into the nematode *Caenorhabditis elegans* leads to specific silencing of genes highly homologous in sequence to the delivered dsRNA (Fire et al., Nature **391** (1998) 806-811). RNAi was also observed subsequently in insects (Kennerdell and Carthew, Cell **95** (1998), 1017-1026), frog (Oelgeschlager et al., Nature **405** (2000), 757-763), and other animals including mice (Svoboda et al., Development **127** (2000), 4147-4156; Wianny and Zemicka-Goetz, Nat. Cell Biol. **2** (2000), 70-75) and is likely to also exist in humans. RNAi is closely linked to the posttranscriptional gene-silencing (PTGS) mechanism of co-suppression in plants and quelling in fungi (Cogoni and Macino, Curr. Opin. Microbiol. **2** (1999), 657-662; Mourrain et al., Cell **101** (2000), 533-542; Smardon et al., Curr. Biol. **10** (2000), 169-178), and some components of the RNAi machinery are also necessary for posttranscriptional silencing by co-suppression (Catalanotto et al., Nature **404** (2000), 245; Demburg et al., Genes & Dev. **14** (2000), 1578-1583; Ketting and Plasterk, Nature **404** (2000), 296-298). The topic has been reviewed recently (Fire, Trends Genet. 15 (1999), 358-363; Sharp, Genes & Dev. **13** (1999), 139-141; Bass, Cell **101** (2000), 235-238; Bosher and Labouesse, Nat. Cell Biol. **2** (2000), E31-E36; Plasterk and Ketting, Curr. Opin. Genet. Dev **10** (2000), 562-567; Sijen and Kooter, Bioassays **22** (2000), 520-531; Plant Molecular Biology **43,** issue 2/3, 2000).

The natural function of RNAi and co-suppression appears to be protection of the genome against invasion by mobile genetic elements such as transposons and viruses, which produce aberrant RNA or dsRNA in the host cell when they become active (Jensen et al., Nat. Genet. **21** (1999), 209-212; Ketting et al., Cell **99** (1999), 133-141; Ratcliff et al., Plant Cell **11** (1999), 1207-1216; Tabara et al., Cell **99** (1999), 123-132; Malinsky et al., Genetics **156** (2000), 1147-1155). Specific mRNA degradation prevents transposon and virus replication, although some viruses are able to overcome or prevent this process by expressing proteins that suppress PTGS (Anandalakshmi et al., Sciences **290** (2000), 142-144; Lucy et al., EMBO J. **19** (2000), 1672-1680; Voinnet et al., Cell **103** (2000), 153-167).

DsRNA triggers the specific degradation of homologous RNAs only within the region of identity with the dsRNA (Zamore et al., Cell **101** (2000), 25-33). The dsRNA is processed to 21-23-nt RNA fragments (Zamore, supra). These short fragments were also detected in extracts prepared from *Drosophila melanogaster* Schneider 2 cells that were transfected with dsRNA before cell lysis (Hammond et al., Nature **404** (2000), 293-296) or after injection of radio-labeled dsRNA into *D. melanogaster* embryos (Yang et al., Curr. Biol. **10** (2000), 1191-1200) or C. *elegans* adults (Parrish et al., Mol. Cell **6** (2000), 1077-1087). RNA molecules of similar size also accumulate in plant tissue that exhibits PTGS (Hamilton and Baulcombe, Sciences **286** (1999), 950-952). It has been suggested that the 21-23-nt fragments are the guide RNAs for target recognition (Hamilton and Baulcombe, Sciences **286** (1999), 950-952; Hammond et al., Nature **404** (2000), 293-296), which is supported by the finding that the target mRNA is cleaved in 21-23-nt intervals (Zamore et al., Cell **101** (2000), 25-33).

Using a *Drosophila* in vitro system, it was demonstrated that 21- and 22-nt RNA fragments are the sequence-specific mediators of RNAi (Elbashir et al., Genes & Dev. **15** (2001), 188-200). The short interfering RNAs (siRNAs) are generated by an RNase III-like enzyme Dicer processing reaction from long dsRNA. Chemically synthesized siRNA duplexes with overhanging 3' ends mediate efficient target RNA cleavage in the lysate, and the cleavage site is located near the center of the region spanned by the guiding siRNA. Elbashir et al. provide evidence that the direction of dsRNA processing determines, whether sense or antisense target RNA can be cleaved by the siRNA-protein complex. Once present, siRNAs trigger the formation of RNA induced silencing complexes (RISC). Helicases in the complex unwind the dsRNA, and the resulting single-stranded RNA (ssRNA) is used as a guide for substrate selection. Once the ssRNA is base-paired with the target mRNA, a nuclease activity, presumably within the complex, degrades the mRNA.

The enzymatic machinery for generating siRNA also appears to be used for the production of a second class of endogenously encoded, small RNA molecules termed microRNAs (miRNAs). miRNA are processed from endogenous transcripts that form hairpin structure, and miRNAs are thought to mediate the translational control of other genes by binding to the 3' ends of their messenger RNAs in animals (Chi et al., Proc Natl Acad Sci U S A. 100 (2003) 6343-6346). miRNAs interfere with expression of messenger RNAs encoding factors that control developmental timing, stem cell maintenance, and other developmental and physiological processes in plants and animals. miRNAs are negative regulators that function as specificity determinants, or guides, within complexes that inhibit protein synthesis (animals) or promote degradation (plants) of mRNA targets (Carrington and Ambros, Science. **301** (2003) 336-338). Plants with altered miRNA metabolism have pleiotropic developmental defects. In *Arabidopsis,* a miRNA has been identified "JAW" that can guide messenger RNA cleavage of several TCP genes controlling leaf development (Palatnik et al., Nature (2003) doi:10.1038/nature01958 (AOP, Published online: 20 August 2003)). Recently, miRNAs were also identified in mouse embryonic stem cells and may play a role in the maintenance of the pluripotent cell state and the regulation of early mammalian development (Houbaviy et al. Dev Cell, 5 (2003), 351-358).

Thus, RNAi seems to be an evolutionary conserved mechanism in plant and animal cells that directs the degradation of mRNA homologous to siRNA. The ability of siRNA to direct gene silencing in mammalian cells has raised the possibility that siRNA might be used to investigate gene function in a high throughput fashion or to modulate gene expression in human diseases.

In US-P-6,506,559 a process for introducing dsRNA into a living cell is disclosed. The objective is to inhibit gene expression by using anti-sense or triple-strand methods. Inhibition is sequence specific in that the nucleotide sequences of the duplex region of the RNA and of a portion of the target gene are identical.

In WO-02/44321 chemically synthesized siRNA duplexes of 19-25 nucleotides with overhanging 3' ends are disclosed to mediate efficient target mRNA cleavage. In WO-03/006477 another method of inducing gene silencing is disclosed. Disclosed are engineered dsRNA precursors which, when expressed in a cell, are processed by the cell to produce siRNAs that selectively silence targeted genes using the cell's own RNAi pathway.

US-P-20020173478 and 20030084471 show the applicability of RNAi to mammalian cells including human cells and cell lines, as well as for administration to human patients.

There is a need in the art to elucidate the biological effect of siRNA in a cell. Also desired is to determine which of the siRNAs formed from one particular gene actually exerts a or the best biological effect.

### Detailed Description of the invention

The present invention provides an easy and fast method for determining the biological impact of a number of siRNA-species on living cells. The method is well adapted for the screening of a large number of genes and a large number of siRNAs for their effect on gene expression in a cell which may be measured by a variety of different parameters.

In particular the present invention provides a method for investigating the biological effect of a siRNA directed against at least one gene present in a cell comprising the steps of, providing a support, containing on pre-determined locations thereon at least one siRNA species; plating cells onto the surface of the array under conditions allowing proliferation of the cells and entry of the siRNA into the cells; optionally exposing the cells to an agent of interest; and detecting the biological impact of the siRNA on the cell.

### Description of the drawings

Fig. 1 shows an embodiment for the screening of siRNA originated from different gene sequences for their effect on living cells. The siRNA have been produced by digestion with a Dicer enzyme of a long double stranded RNA and the released fragments immobilized on an array before transfection into the cells and detection of a signal related to a searched effect.

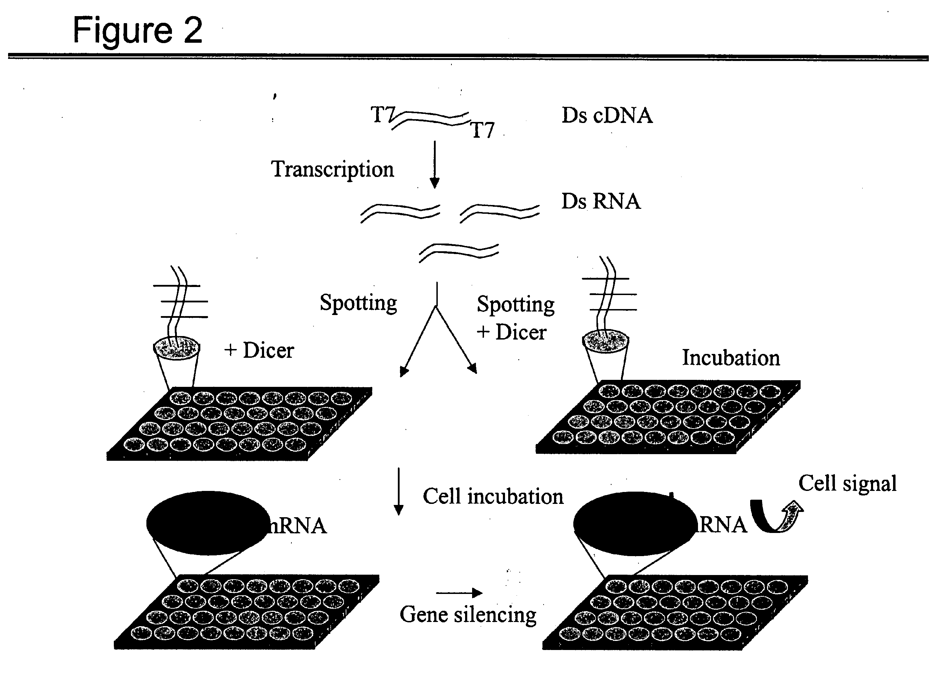

Fig. 2 shows an embodiment for the screening of siRNA originated from different gene sequences for their effect into living cells. The dsRNA have been immobilized on a support before being digested with a Dicer enzyme and transfected into the cells.

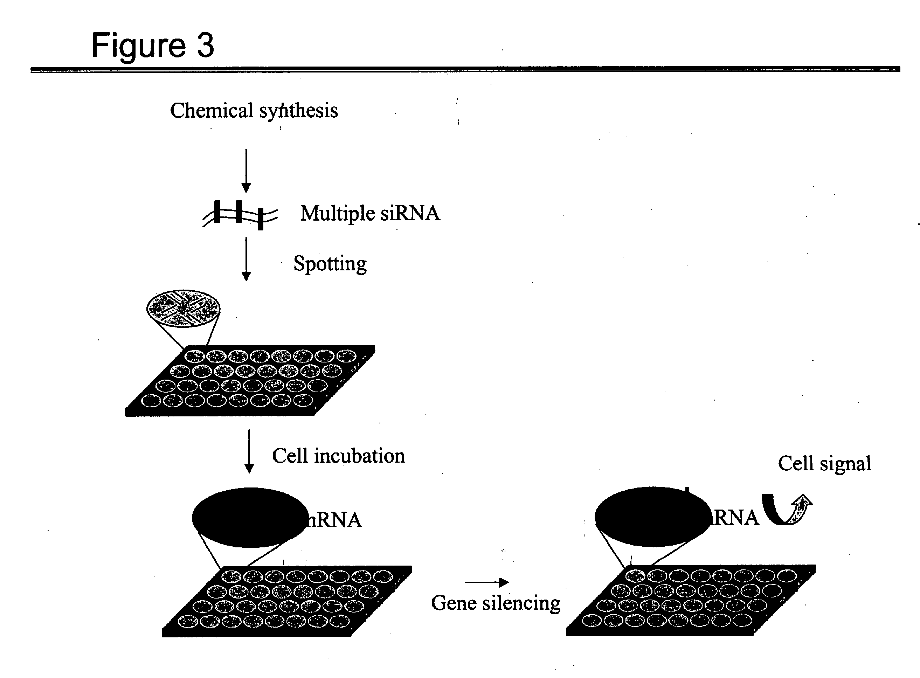

Fig. 3 shows an embodiment for the screening of multiple siRNA for their effect into living cells. The siRNA have been produced by chemical synthesis and immobilized on an array before transfection into the cells.

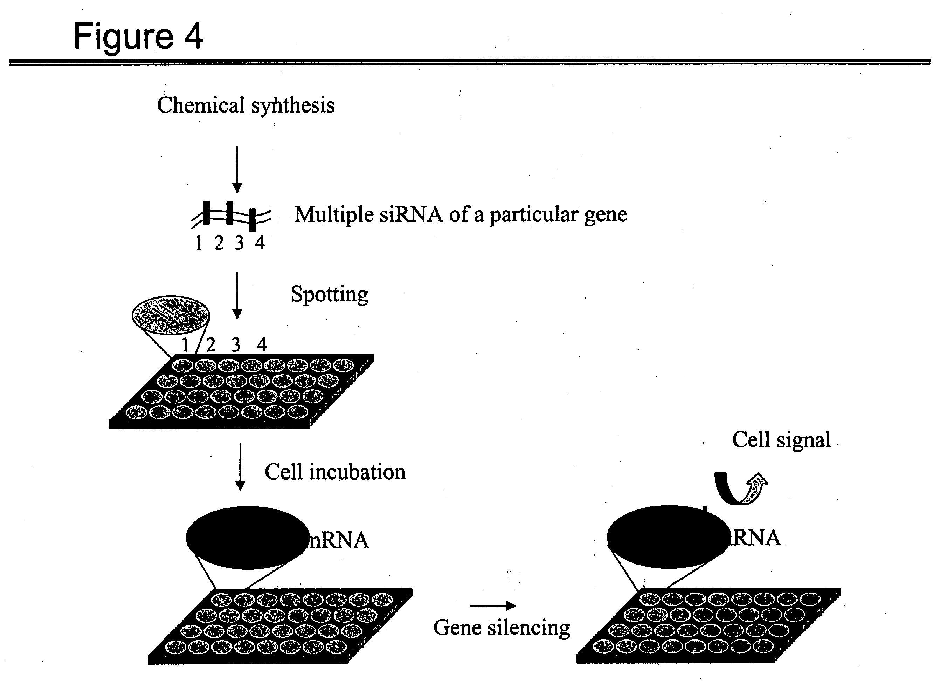

Fig. 4 shows an embodiment for the screening of multiple siRNA corresponding to different parts of the sequence of a particular gene. The siRNA have been produced by chemical synthesis and individual siRNAs are immobilized on an array at different locations before transfection into the cells.

### Definitions

The term "genes" shall designate the genomic DNA which is transcribed into an RNA species, preferably into an mRNA and then translated into a peptide or protein.

The term "nucleotide" as used herein refers to nucleotides present in nucleic acids (either DNA or RNA) compared with the bases of said nucleic acid, and includes nucleotides comprising usual or modified bases.

References to nucleotide(s), oligonucleotide(s), polynucleotide(s) and the like include analogous species wherein the sugar-phosphate backbone is modified and/or replaced, provided that its hybridization properties are not destroyed. By way of example, the backbone may be replaced by an equivalent synthetic peptide, called Peptide Nucleic Acid (PNA).

Dicer is an endoribonuclease containing a RNase III domain and is the enzyme responsible for cleavage of long dsRNA into siRNA. The siRNA produced by the Dicer are 21 to 23 base long and contain a 3'dinucleotide overhangs with 5'-phosphate and 3'-hydroxyl terminus (cf. Bernstein et al., Nature 409 (2001), 363-366).

The term siRNA (short interfering RNA) relates to a double stranded RNA having the sequence of one of the two complementary strands at least in part identical to a part of the sequence of the gene. siRNA also incorporates shRNA (short hairpin RNA) and miRNA (microRNA).

The term "location" shall designate a spot on the support, which is represented by a discrete region on the support spaced apart from another location.

### Detailed description of the invention

The main characteristic of the invention is to obtain a direct analysis and an overview of siRNAs which affect cellular regulation after transfection of siRNA present on a support in the form of array into living cells. The invention is not limited by the number of siRNA to be screened. The array allows the binding of either from 2 to 100 and more preferably until 1000 features and thus provide the siRNA to be tested in cell culture.

The present method makes use of a support, which may have the form of a culture plate, a multi-well plate, glasses, slides, discs or beads and may be prepared from any material conventionally used for this purpose, such as plastics, glass, silicon or agar. The support should be of a material coated or not with external molecules in order to be compatible with the presence of living cells. Supports such as plastic, being polystyrene, polypropylene, polymethylmethacrylate or other polymers treated for cell adhesion are preferred. Preferably, the support is a plastic support having a hydrophilic three dimensional structure or is covered with a substrate having such a structure. According to a preferred embodiment dextran or polyethylene glycol polymers grafted on the support are present. In another embodiment, hydrophilic proteins such as gelatin or collagen or fibronectin are used for the coating of the support.

On the support, different siRNA-species are arranged on predetermined locations thereof.

In principle, this may be attained by either spotting the siRNA species on the support itself, or by spotting a molecule on the support, from which the siRNA may be synthesized at a later stage.

According to a first embodiment, the different siRNAs are synthesized prior to spotting them on the support by first producing double stranded RNAs having the sequence of either the full length of a mRNA, or the coding part thereof or a part of the coding part of a specific gene to be investigated.

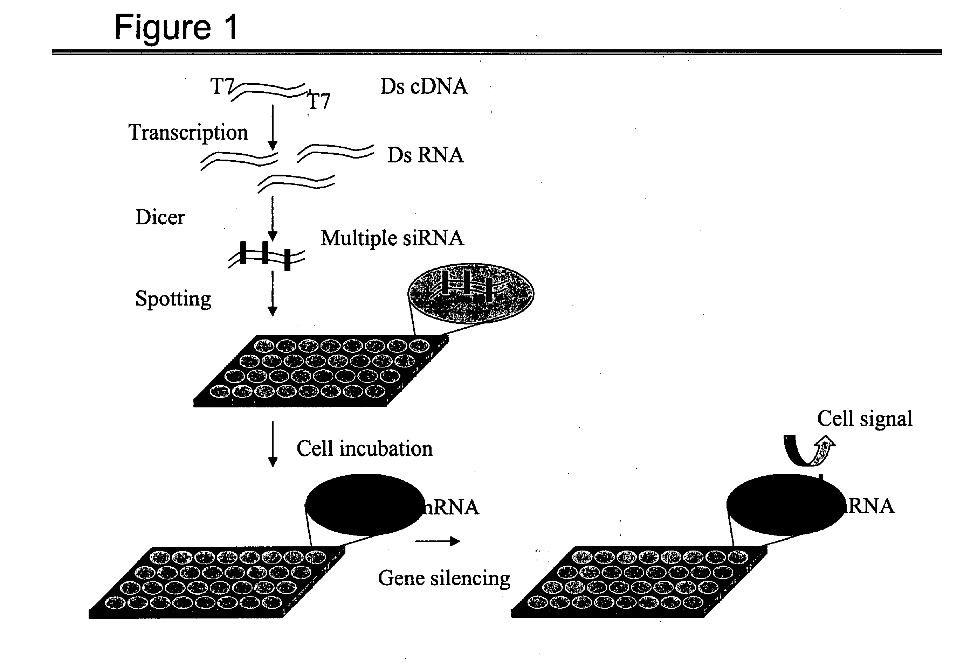

Double stranded RNA may be obtained e.g. by amplification of a gene via PCR using primers having a T7 promotor sequence linked thereto and subsequent transcription of the two strands by copying using the T7 polymerase. The two RNA strands are then allowed to anneal in solution in order to form a double strand RNA with a sequence corresponding to the initial gene (cf. Fig. 1).

Starting from a dsRNA obtained e.g. as described above, the siRNA may be formed by enzymatic digestion using the DICER enzymes for cleavage. Cleavage of the_dsRNA may also be obtained with the reconstituted RNase III or other enzymes as long as they also lead to the formation of the siRNA pieces from the dsRNA.

In case the entire gene sequence, either on the DNA or the RNA level, has been used for the preparation of siRNA, the siRNA-species/-pool to be spotted will contain all of the different siRNA-molecules derived from the initial gene sequence. Thus, one spot will give an indication about the effect of one or more siRNA-molecules contained in the siRNA-pool produced.

However, if only a part of the gene of has been utilized for the preparation of the siRNA, it may be evaluated, whether the siRNA-species/-pool derived from a particular part of the gene has a greater or lesser impact on a biological effect. In such a situation several locations/spots may be used for the analysis of the impact of the different siRNAs formed from different parts of the sequence of the gene on cells. The identification of a biological effect on one of these locations will provide evidence for the effect of an siRNA-species derived from this gene. The effect occurring on such particular location will also indicate the sequence part from which the siRNA has been produced. Accordingly, the invention is also well suited for the determination of one or more specific parts of a gene from which an siRNA originates.

The different siRNA-species thus obtained and derived from different genes or different parts of the genes are then spotted onto the surface of the support on predetermined locations to form an array.

The siRNA may be deposited by a robot based micro-arrayer. The liquid containing the siRNA is deposited on the surface either by a contact or non-contact spotting process. Preferably, the process of liquid deposit for micro-array production is used in this invention with different methods of spotting liquid on surfaces being described in the Manual D. Bowtell and J. Sambrook ( DNA Microarrays , Cold Spring Harbor Laboratory Press, Cold Spring Harbor, New York, 2003). The liquid may also be deposited by pipetting the liquid onto the surface when large surfaces locations have to be covered or when the surface is divided into physically or chemically separate locations.

According to an alternative embodiment, the different siRNAs are prepared on the predetermined locations of the support/array itself. This may be achieved by first attaching a starting nucleic acid on the support, e.g. the dsRNA as mentioned above, and then producing siRNA therefrom, the effect of which shall be elucidated, by subjecting it to an enzymatic degradation as mentioned above. Thus, the different locations on the support contain different specific double stranded RNA. The RNA, subjected to digestion by e.g. DICER applied onto the surface of the support, is incubated for a particular time period, so as to obtain the corresponding siRNAs originating from the double stranded RNA being specific at each location.

According to yet an alternative embodiment, the siRNAs specific for a gene may also be synthesized chemically and deposited at/on the respective specific locations on a support.

According to still another embodiment, short (21-23-base) siRNAs with 2-base 3' overhangs may be produced by in vitro transcription according to the method siLentGene (Promega, Madison, WI, USA). A gene sequence may be attached to an engineered U6 promotor and terminator after PCR amplification. This design enables transcription of precise sense and antisense RNA with the recombinant 3' additions. The two strands are then allowed to anneal to form a double RNA strand. The terminator consists of a short stretch of uridines; this is compatible with the original siRNA design that terminates with a two uridine 3' overhang (Farrell et al, Cell 11 (1977), 187-200).

In an alternative embodiment, the siRNAs may be processed from short hairpin RNA (shRNA). shRNA can be produced by means of pGE-1 shRNA mammalian expression vector (Stratagene, La Jolla, CA, USA). The shRNA is generated from an RNA transcript that consists of sense and antisense strands separated by a loop sequence. The RNA transcript folds back on itself to form an hairpin. The pGE-1 expression vector uses a human U6 promotor, an RNA polymerase III promoter, to generate shRNA in mammalian cells. The U6 promotor is relatively short and uses a short polyU stretch as a terminator sequence.

Alternatively, shRNA can be produced following the method provided by AmpliScribe T7-Flash Transcription (Epicentre Madison, WI, USA). Two complementary 87-base DNA oligonucleotides are synthesized to serve as the in vitro transcription templates to produce shRNA The first oligonucleotide contains a T7 RNA polymerase promoter sequence followed by a 29-base sequence complementary to the target messenger RNA, an 8-base non-complementary sequence (hairpin) and the complement to the 29-base sequence. The second oligonucleotide is the complement of the first. The two oligonucleotides are denatured by heating at 95°C for 5 min and annealed for 10 min at 65°C, followed by 37°C for 10 min. After in vitro transcription for 30 min, 66-base shRNA are produced, treated with DNase I and phenol : chloroform extracted.

The different locations on the support are preferably separated by a barrier, which may be a physical barrier or a chemical barrier. Examples for physical barriers are wells, e.g. the support being a 96, 384, 1536 multi-well plate, thus creating separated locations onto which siRNA maybe spotted individually. 384-well and 1536-well plates are available from BD Falcon for cell based assays (Merck Eurolab sa, Leuven, Belgium) or from Nunc A/S (Roskilde, Denmark). 6144 format microtiter plates are available from Parallel Synthesis Technologies Inc. (PSTI, Menlo Park, CA, USA). Other physical barriers are tubes such as 96, 384, 1536 or even 6144 tubes deposit at the surface of the support. Tubes are similar to the well formats but do not have a plain bottom sot that when deposit on the surface of the support, they create locations isolated from each other. An examples for a chemical barrier is e.g. described in DE 0019949735A1, where defined areas within a hydrophobic surface are provided with hydrophilic anchors allowing the precise location and confinement of solutions containing various biomolecules, describing usefulness for DNA micro-arrays.

After fixation of the RNAs on the support, the surface is carefully washed and the cells are plated thereon. As the cells to be investigated any of the known type of cells are envisaged, e.g., embryonic stem cells, cardiomyocytes, endothelial cells, sensory neurons, motor neurons, CNS neurons, astrocytes, glial cells, Schwann cells, mast cells, eosinophils, smooth muscle cells, skeletal muscle cells, pericytes, lymphocytes, tumor cells, monocytes, macrophages, foamy macrophages, melanocytes, granulocytes, keratinocytes, synovial cells, fibroblasts and epithelial cells.

The cells are preferably kept in a medium allowing proliferation and/or growth and/or propagation thereof on the support. In addition, the said medium should also allow or even facilitate incorporation of the multiplicity of siRNAs into the cells. Examples for appropriate media are based on cationic liposomal and polyamine based reagents. The choice of reagent and the conditions under which it is used depend on the type of cells and the type of molecule being transfected. Reagents optimized for delivery of plasmid DNA are not optimal for delivering siRNA. siPort Amine (a polyamine mixture) or siPort Lipid (a mixture of cationic and neutral lipids) Transfection Agent of Ambion (Austin, Tx., USA) are the transfection agents of choice for introducing siRNAs into a wide variety of mammalian cultured cells. Different cell types are preferentially transfected with one or the other transfection agent. Both agents demonstrate low toxicity and are ideal for introducing individual siRNAs prepared by chemical synthesis or in vitro transcription, as well as siRNA populations prepared by digestion of long dsRNA by RNase III or Dicer being preferred.

After a time period of culture, e.g. 3 - 6 hrs, a transfection agent is added to the culture and the cells are incubated for another 3 to 6 hrs, allowing the siRNA molecules to enter the cells.

Transfection efficiency is dependent on the nature of the cells, the density of plating but also of the transfected material. siRNA are preferably transfected using either cationic liposomal or polyamine based molecules (Demeterco et al, J. Clin. Endocrinol. metab. 87 (2002 ), 3475-3485; Harbort et al., J. Cell Science **114** (2001), 4557-4565).

The cells are then incubated on the support for a time period, sufficient for the siRNA to exert a or their biological effect, e.g. of from about 6 to 72 hours, preferably of from 12 to 48 hours, more preferably of from 12 to 24 hours, under conditions allowing proliferation. Proliferation in the sense of the present application means that at least cellular activities are allowed to continue. Thus, it is not necessary that the cells grow or multiply, but only that the biological effect of the siRNA at a certain level, i.e. either on the RNA level or the protein level or eventually on the level of a phenotypic appearance may be observed.

After incubation, the cells are assayed for the biological effect of interest, which may be related to e.g. cell division, proliferation, apoptosis or cell differentiation. In principle, this assaying step may be performed on the phenotypic level, the protein level and the RNA level with methods such as but not limited to the immunolabeling, RT-PCR, Northern, Western analysis, enzymatic activities, DNA probe labeling (FISH), gene expression micro-array analysis..

According to a preferred embodiment, the cells plated on the location of the siRNA and exposed to the siRNA are removed and the reduction in the level of the mRNA for a particular gene is determined by real time RT-PCR using primers specific for the particular gene, from which the siRNAs at this location have been derived. Alternatively, a lysing solution may be introduced into the confined area of the particular location so as to obtain a cell extract on which the analysis is performed. A comparison of the level of a particular gene in one location bearing the siRNA with other control locations without siRNA gives the values of the efficacy of the RNAi present at this location.

The particular location of the cells showing a biological change is then identified and correlated with the gene from which the siRNAs were produced. The correlation is then made between the gene associated siRNA and the biological effect. Also, the transcription and/or translation of a gene, from which the siRNAs have been derived may have an influence on other genes, which the present invention allows to elucidate. Generally, the amount of mRNA of another gene may be increased and/or decreased due to inhibition of the gene of interest eventually leading to a biological phenotypic effect. Hence the present invention enables a look into the network of cellular activities and the impact of the transcription of one gene on another gene. The same applies, in case the cells have been contacted prior to transfection with the siRNA with an agent, e.g. a potential drug. Here the impact of the siRNA on the effect of the chemical compound on the cell may be elucidated.

The present method is well suited for screening various siRNAs corresponding to different parts of the sequence of a particular gene. So, the method provides a way for the identification of the different siRNAs from one particular gene which gives the best effect.

The invention also provides a novel method for the transfection of cells with multiple RNAi with possible multiple transfections for multiple genes and the identification and the correlation between the related cell behavior with the given gene affected by the RNAi or with a particular RNAi and the tested biological effect.

In an embodiment, the array also contains a positive transfection control, which may be a DNA sequence coding for any detectable protein, such as enzymes, preferably the Green Fluorescent Protein (GFT ), which upon transfection allows an easy detection of the transfected gene by fluorescence measurement.

The method and support/micro-array as described herein may be utilized as part of a diagnostic and/or quantification kit which comprises means and media for performing analysis of siRNA being present on a support and the necessary reagent and solutions for the transfection of the siRNA into living cells and eventually tools for determining the biological effect of the siRNA on the cells.

Also provided by the present invention is a kit for screening of multiple siRNAs for their impact on cells or daughter cells having been transfected by siRNA according to the present invention and testing for a biological parameter change related to a specific gene expression.

Also provided by the present invention is a kit for the screening of a compound on the efficiency on specific gene expression after their transfection of siRNA into living cells, said kit comprises a solid support with the attached siRNA having sequences identical or complementary to the gene to be targeted.

The following example is intended to illustrate the invention without limiting it thereto.

### Example

Preparation of the siRNA related to a gene

The preparation of dsRNA was performed using the T7 RiboMAX Express RNAi system (Promega, Madison, WI, USA).

The cloned genes were amplified with specific primers, one of them having a T7 promoter of the type 5'-TAATACGACTCACTATAGGN (primer) 3'. Two clones were used one coding for the Ornithine decarboxylase1 (ODC) and the other one for Bcl-2. The specific primers were
5'-AGAGGAACAGGATGCCAGC-3', and
5'-TTAAAAACAGGTCACAACACTAA-3'
for the ODC,
5'-AGCACAGAAGATGGGAACAC-3', and
5'-CAGCCACCTCTTAAAAGTATC-3'
for bcl-2.

The resulting amplicons had a T7 promoter on one strand thus allowing the transcription of this strand. The transcription was performed with the RiboMAX Express as proposed by the manufacturer by incubating each of the T7 stranded sequences with the transcriptase. The reaction was performed at 37°C for 30 min. After transcription, the DNA template is removed by digestion with DNase, being RNase free. The annealing of the two RNA strands was obtained by incubating an equal concentration and volume of the two RNA strands for 10 min at 70°C. The single stranded RNA was then removed by incubation with RNase which digests the single stranded RNA. The double stranded RNA was then purified by precipitation in cold ethanol (- 70 °C) and recovered after centrifugation in solution ready for digestion with the Silencer siRNA cocktail kit of Ambion (Austin, TX USA). The double stranded RNA was then digested using 1 U of RNase III per microgram of RNA with incubation of 1 h at 37°C. After one hour digestion, the double stranded RNA was reduced into small fragments of lower than 30 bp with a majority of between 12 and 15 bp.

The siRNA was transferred in the wells of a 96 well plate and CHO cells were transferred into the wells with the siPort Amine Transfection Agent of Ambion (Austin, Tx., USA) using the experimental protocol provided by the manufacturer with a concentration of 3 µl of transfection agent per cm² of plate surface and a density of cells being at 10⁵ cell per cm².

After 2 days the mRNA of the cells was collected and the level of expression of the two genes tested by real time PCR using the specific primers for the two mRNA.

It could be shown that the level of the mRNA coding for Bcl-2 was lower in the cells which have been transfected in the wells containing the siRNA specific for the Bcl-2 sequence.

## Claims

1. A method for investigating the biological effect of a siRNA directed against at least one gene present in a cell comprising the steps of:
providing a support, containing on pre-determined locations thereon at least one siRNA species;
plating cells onto the surface of the array under conditions allowing proliferation of the cells and entry of the siRNA into the cells;
optionally exposing the cells to an agent of interest; and
detecting the biological impact of the siRNA on the cell.

2. The method according to claim 1, wherein the siRNA present at the predetermined locations on the support is obtained by enzymatic digestion of double stranded nucleotides or by chemical synthesis.

3. The method according to claim 2, wherein the enzymatic digestion of the double stranded nucleotides is performed at the predetermined locations of the surface of the support.

4. The method according to any of the claims 2 to 3, wherein the double stranded nucleotides are RNA copies corresponding to a partial or complete coding sequence or the essentially full-length mRNA of the corresponding genes.

5. The method according to any of the claims 2 to 3 wherein the double stranded RNA nucleotides are short hairpin RNA (shRNA).

6. The method according to any of the claims 2 to 3 wherein the double stranded RNA nucleotides are microRNA (miRNA).

7. The method according to any of the claim 2, wherein the enzymatic digestion is performed on the double stranded RNA nucleotides by DICER or other RNase III-type enzymes.

8. The method according to any of the claims 1 to 7, wherein the support is selected from the group consisting of a culture plate, multi-well plate, glasses, slides, discs, or beads.

9. The method according to any of the preceding claims, wherein the predetermined locations for the siRNA are isolated from each other by a physical barrier.

10. The method according to claim 9, wherein the barriers are tubes or a chemical barrier.

11. The method according to any of the preceding claims, wherein the biological impact of the siRNA on the cells is tested by determining biological parameters linked to cell division, cell proliferation, apoptosis and/or cell differentiation.

12. The method according to any of the claims 1 to 11, wherein specific proteins of the cells are analyzed by means of Western analysis.

13. The method according to any of the claims 1 to 11, wherein enzymatic activities on the specific location are assayed.

14. The method according to any of the claims 1 to 11, wherein one or more specific mRNAs of the cells are analyzed with a northern analysis.

15. The method according to any of the claims 1 to 11, wherein the step of determining the biological impact of the siRNA includes performing a RT-PCR for quantifying a transcript of particular gene of the cells being affected by the presence of the siRNA present in the location.

16. The method according to any of the preceding claims, for the detection and/or identification of the biological effect of a chemical compound on a cell.

17. The method according to any of the preceding claims, wherein the biological effect is quantified using a real-time detection method.

18. A micro-array comprising a support, containing on predetermined locations thereof an siRNA.

19. A kit for screening of multiple siRNAs for their impact on cells or daughter cells having been transfected by siRNA and testing for a biological parameter change related to a specific gene expression, said kit comprising a micro-array according to claim 15.
